# EUROPEAN PATENT APPLICATION

(11) **EP 3 878 467 A1**
(43) Date of publication of application: **15.09.2021**
(21) Application number: 19878080.1
(22) Date of filing: 30.10.2019
(51) Int. Cl.: A61K 39/395, C07K 16/40, A61K 47/00

(54) **STABLE FORMULATION CONTAINING ANTI-PCSK9 ANTIBODY**

(30) Priority: 31.10.2018 CN 201811283440
(71) Applicant: Shanghai Junshi Biosciences Co., Ltd., Shanghai 201210 (CN); Suzhou Junmeng Biosciences Co., Ltd., Jiangsu 215002 (CN)
(72) Inventor: LIU, Hongchuan, Suzhou, Jiangsu 215002 (CN); LIU, Peixiang, Suzhou, Jiangsu 215002 (CN); ZHANG, Jing, Suzhou, Jiangsu 215002 (CN); DING, Shenhao, Suzhou, Jiangsu 215002 (CN); ZHANG, Mei, Suzhou, Jiangsu 215002 (CN); WU, Chun, Suzhou, Jiangsu 215002 (CN); FENG, Hui, Suzhou, Jiangsu 215002 (CN); WU, Hai, Shanghai 201210 (CN)
(74) Representative: V.O.
(86) International application number: PCT/CN2019/114233
(87) International publication number: WO 2020/088492

(57) **Abstract**

The present invention provides a highly concentrated liquid formulation comprising an anti-PCSK9 (human proprotein convertase subtilisin/kexin 9) antibody. The formulation further comprises a buffer, a stabilizer and a surfactant. The liquid formulation disclosed has low viscosity, and the antibody is highly stable after several months of storage.

Sequence Listing

<110>  SHANGHAI JUNSHI BIOSCIENCES CO., LTD.
       SUZHOU JUNMENG BIOSCIENCES CO., LTD.

<120>  STABLE PREPARATION COMPRISING ANTI-PCSK9 ANTIBODY

<130>  198719PCWO

<150>  CN 201811283440.7
<151>  2019-10-31

<160>  10

<170>  SIPOSequenceListing 1.0

<210>  1
<211>  5
<212>  PRT
<213>  Artificial Sequence

<220>
<223>  HCDR1

<400>  1
Ser Tyr Gly Ile His
1               5

<210>  2
<211>  16
<212>  PRT
<213>  Artificial Sequence

<220>
<223>  HCDR2

<400>  2
Val Ile Trp Arg Gly Gly Ile Thr Asp Tyr Asn Ala Pro Phe Met Ser
1               5                   10                  15

<210>  3
<211>  3
<212>  PRT
<213>  Artificial Sequence

<220>
<223>  HCDR3

<400>  3
His Arg Asp
1

<210>  4
<211>  11
<212>  PRT
<213>  Artificial Sequence

<220>
<223>  LCDR1

<400>  4
Gln Ala Ser Gln Asp Ile Asn Lys Tyr Ile Asp
1               5                   10

<210>  5
<211>  7
<212>  PRT
<213>  Artificial Sequence

<220>
<223>  LCDR2

<400>  5
Tyr Ala Ser Thr Leu Gln Pro
1               5

<210>  6
<211>  8
<212>  PRT
<213>  Artificial Sequence

<220>
<223>  LCDR3

<400>  6
Leu Gln Tyr Asp Asp Leu Trp Thr
1               5

<210>  7
<211>  111
<212>  PRT
<213>  Artificial Sequence

<220>
<223>  Heavy chain variable region

<400>  7
Gln Val Gln Leu Gln Glu Ser Gly Pro Gly Leu Val Lys Pro Ser Gln
1               5                   10                  15
Thr Leu Ser Leu Thr Cys Thr Val Ser Gly Phe Ser Ile Ser Ser Tyr
            20                  25                  30
Gly Ile His Trp Ile Arg Gln Ser Pro Gly Lys Gly Leu Glu Trp Ile
        35                  40                  45
Gly Val Ile Trp Arg Gly Gly Ile Thr Asp Tyr Asn Ala Pro Phe Met
    50                  55                  60
Ser Arg Val Thr Ile Ser Lys Asp Asn Ser Lys Asn Gln Val Ser Phe
65                  70                  75                  80
Lys Leu Ser Ser Val Thr Ala Ala Asp Thr Ala Val Tyr Tyr Cys Ala
                85                  90                  95
Asn His Arg Asp Trp Gly Gln Gly Thr Leu Val Thr Val Ser Ser
            100                 105                 110

<210>  8
<211>  106
<212>  PRT
<213>  Artificial Sequence

<220>
<223>  Light chain variable region

<400>  8
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asp Ile Asn Lys Tyr
            20                  25                  30
Ile Asp Trp Tyr Gln His Lys Pro Gly Lys Ala Pro Lys Leu Leu Ile

35                  40                  45
His Tyr Ala Ser Thr Leu Gln Pro Gly Val Pro Ser Arg Phe Ser Gly
    50                  55                  60
Ser Gly Ser Gly Arg Asp Tyr Thr Phe Thr Ile Ser Ser Leu Gln Pro
65                  70                  75                  80
Glu Asp Ile Ala Thr Tyr Tyr Cys Leu Gln Tyr Asp Asp Leu Trp Thr
                85                  90                  95
Phe Gly Gln Gly Thr Lys Val Glu Ile Lys
            100                 105

<210>  9
<211>  438
<212>  PRT
<213>  Artificial Sequence

<220>
<223>  Heavy Chain

<400>  9
Gln Val Gln Leu Gln Glu Ser Gly Pro Gly Leu Val Lys Pro Ser Gln
1               5                   10                  15
Thr Leu Ser Leu Thr Cys Thr Val Ser Gly Phe Ser Ile Ser Ser Tyr
            20                  25                  30
Gly Ile His Trp Ile Arg Gln Ser Pro Gly Lys Gly Leu Glu Trp Ile
        35                  40                  45
Gly Val Ile Trp Arg Gly Gly Ile Thr Asp Tyr Asn Ala Pro Phe Met
    50                  55                  60
Ser Arg Val Thr Ile Ser Lys Asp Asn Ser Lys Asn Gln Val Ser Phe
65                  70                  75                  80
Lys Leu Ser Ser Val Thr Ala Ala Asp Thr Ala Val Tyr Tyr Cys Ala
                85                  90                  95
Asn His Arg Asp Trp Gly Gln Gly Thr Leu Val Thr Val Ser Ser Ala
            100                 105                 110
Ser Thr Lys Gly Pro Ser Val Phe Pro Leu Ala Pro Cys Ser Arg Ser
        115                 120                 125
Thr Ser Glu Ser Thr Ala Ala Leu Gly Cys Leu Val Lys Asp Tyr Phe
    130                 135                 140
Pro Glu Pro Val Thr Val Ser Trp Asn Ser Gly Ala Leu Thr Ser Gly
145                 150                 155                 160
Val His Thr Phe Pro Ala Val Leu Gln Ser Ser Gly Leu Tyr Ser Leu
                165                 170                 175
Ser Ser Val Val Thr Val Pro Ser Ser Ser Leu Gly Thr Lys Thr Tyr
            180                 185                 190
Thr Cys Asn Val Asp His Lys Pro Ser Asn Thr Lys Val Asp Lys Arg
        195                 200                 205
Val Glu Ser Lys Tyr Gly Pro Pro Cys Pro Pro Cys Pro Ala Pro Glu
    210                 215                 220
Phe Leu Gly Gly Pro Ser Val Phe Leu Phe Pro Pro Lys Pro Lys Asp
225                 230                 235                 240
Thr Leu Met Ile Ser Arg Thr Pro Glu Val Thr Cys Val Val Val Asp
                245                 250                 255
Val Ser Gln Glu Asp Pro Glu Val Gln Phe Asn Trp Tyr Val Asp Gly
            260                 265                 270
Val Glu Val His Asn Ala Lys Thr Lys Pro Arg Glu Glu Gln Phe Asn
        275                 280                 285
Ser Thr Tyr Arg Val Val Ser Val Leu Thr Val Leu His Gln Asp Trp
    290                 295                 300
Leu Asn Gly Lys Glu Tyr Lys Cys Lys Val Ser Asn Lys Gly Leu Pro
305                 310                 315                 320
Ser Ser Ile Glu Lys Thr Ile Ser Lys Ala Lys Gly Gln Pro Arg Glu
                325                 330                 335
Pro Gln Val Tyr Thr Leu Pro Pro Ser Gln Glu Glu Met Thr Lys Asn
            340                 345                 350

Gln Val Ser Leu Thr Cys Leu Val Lys Gly Phe Tyr Pro Ser Asp Ile
        355                 360                 365
Ala Val Glu Trp Glu Ser Asn Gly Gln Pro Glu Asn Asn Tyr Lys Thr
    370                 375                 380
Thr Pro Pro Val Leu Asp Ser Asp Gly Ser Phe Phe Leu Tyr Ser Arg
385                 390                 395                 400
Leu Thr Val Asp Lys Ser Arg Trp Gln Glu Gly Asn Val Phe Ser Cys
                405                 410                 415
Ser Val Met His Glu Ala Leu His Asn His Tyr Thr Gln Lys Ser Leu
            420                 425                 430
Ser Leu Ser Leu Gly Lys
        435

<210>  10
<211>  213
<212>  PRT
<213>  Artificial Sequence

<220>
<223>  Light chain

<400>  10
Asp Ile Gln Met Thr Gln Ser Pro Ser Ser Leu Ser Ala Ser Val Gly
1               5                   10                  15
Asp Arg Val Thr Ile Thr Cys Gln Ala Ser Gln Asp Ile Asn Lys Tyr
            20                  25                  30
Ile Asp Trp Tyr Gln His Lys Pro Gly Lys Ala Pro Lys Leu Leu Ile
        35                  40                  45
His Tyr Ala Ser Thr Leu Gln Pro Gly Val Pro Ser Arg Phe Ser Gly
    50                  55                  60
Ser Gly Ser Gly Arg Asp Tyr Thr Phe Thr Ile Ser Ser Leu Gln Pro
65                  70                  75                  80
Glu Asp Ile Ala Thr Tyr Tyr Cys Leu Gln Tyr Asp Asp Leu Trp Thr
                85                  90                  95
Phe Gly Gln Gly Thr Lys Val Glu Ile Lys Arg Thr Val Ala Ala Pro
            100                 105                 110
Ser Val Phe Ile Phe Pro Pro Ser Asp Glu Gln Leu Lys Ser Gly Thr
        115                 120                 125
Ala Ser Val Val Cys Leu Leu Asn Asn Phe Tyr Pro Arg Glu Ala Lys
    130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
Cys Glu Val Thr His Gln Gly Leu Ser Ser Pro Val Thr Lys Ser Phe
        195                 200                 205
Asn Arg Gly Glu Cys
    210

## Description

### TECHNICAL FIELD

The present invention relates to the field of therapeutic antibody formulations, and particularly, to the field of pharmaceutics, wherein the formulation comprises a humanized antibody that specifically binds to human proprotein convertase subtilisin/kexin type 9 (PCSK9).

### BACKGROUND

Proprotein convertase subtilisin/kexin type 9 (PCSK9) is a proprotein convertase which promotes the degradation of low-density lipoprotein (LDL) receptors on liver cell surface, thereby increasing the LDL cholesterol level in plasma. Its expression is highly associated with human dyslipidemia and cardiovascular-related diseases. Patent Publication No. WO2017088782 discloses various anti-PCSK9 antibodies that can significantly reduce LDL level in blood by antagonizing PCSK9 bioactivity which are promising in treating hypercholesterolemia and other related diseases. Like any protein therapeutics, therapeutic anti-PCSK9 antibodies are subject to physical and chemical instabilities, such as aggregation, denaturation, cross-linking, deamidation, isomerization, oxidation, and shearing during manufacture or storage (Wang et al., J. Pharm. Sci., 96:1-26, 2007). Therefore, it is very challenging to develop an antibody formulation with stable physicochemical properties.

Hypercholesterolemia and other related diseases are generally chronic diseases. Thus, it is important to provide patients with convenience for out-of-hospital or self drug administration. Generally, protein therapeutics can only be administered parenterally, among which subcutaneous (SC) or intramuscular (IM) administrations can be more cost-efficient and can improve the convenience for patients and healthcare providers during administration. However, small volume required by SC or IM injections (typically 0.5 to 2 mL) raises an additional challenge of formulation, since such administration requires higher-concentration of antibody formulations, typically between 100 mg to 1 g protein per dose to achieve a therapeutic purpose. However, highly concentrated protein formulations are often accompanied by risks such as protein aggregation, precipitation and increased viscosity, which may result in negative consequence during process, manufacture and storage. Increased viscosity can also have adverse effects on administration of the formulation, such as feelings of pain and burn, and limitations on selections of drug delivery device (Shire et al., J. Pharm. Sci., 93:1390-1402, 2004).

Thus, there is a need in the art for highly concentrated protein formulations that particularly provide low viscosity and relieve pain of patients.

### SUMMARY OF INVENTION

The present invention provides a pharmaceutical formulation, comprising a humanized antibody that specifically binds to human proprotein convertase subtilisin/kexin type 9 (PCSK9).

In one aspect, the present invention provides a stable antibody formulation comprising: (1) a buffer; (2) a stabilizer; and (3) an anti-PCSK9 antibody or an antigen-binding fragment thereof.

In one preferred embodiment, the stable antibody formulation further comprises a nonionic surfactant.

In one embodiment, the buffer is a histidine buffer. In one embodiment, the histidine buffer is prepared by L-histidine and L-histidine monohydrochloride. In one embodiment, the concentration of the histidine buffer is about 20 nM.

In one embodiment, the stabilizer comprises one or more selected from arginine or a salt thereof, sorbitol, mannitol, and sucrose. In one embodiment, the stabilizer is an arginine salt. In one embodiment, the concentration of arginine or the salt thereof is about 50 mM to about 200 mM. In some specific embodiments, the concentration of arginine or the salt thereof is about 60 mM, 130 mM, 160 mM, or 165 mM.

In one embodiment, the antibody or the antigen-binding fragment thereof comprises HCDR1, HCDR2, HCDR3, LCDR1, LCDR2 and LCDR3, wherein, HCDR1 has an amino acid sequence of SEQ ID NO: 1; HCDR2 has an amino acid sequence of SEQ ID NO: 2; HCDR3 has an amino acid sequence of SEQ ID NO: 3; LCDR1 has an amino acid sequence of SEQ ID NO: 4; LCDR2 has an amino acid sequence of SEQ ID NO: 5; and LCDR3 has the amino acid sequence SEQ ID NO: 6. In one specific embodiment, the antibody or the antigen-binding fragment thereof comprises a heavy chain variable region (VH) having an amino acid sequence of SEQ ID NO: 7 and a light chain variable region (VL) having an amino acid sequence of SEQ ID NO: 8. In one specific embodiment, the antibody or the antigen-binding fragment thereof comprises a heavy chain (HC) having an amino acid sequence of SEQ ID NO: 9 and a light chain (LC) having an amino acid sequence of SEQ ID NO: 10. In one embodiment, the concentration of the antibody or the antigen-binding fragment thereof is about 100 mg/mL to about 200 mg/mL. In one specific embodiment, the concentration of the antibody or the antigen-binding fragment thereof is about 150 mg/mL.

In one embodiment, the antibody formulation disclosed herein comprises polysorbate 20 or polysorbate 80 at about 0.01% to about 0.05%. In one specific embodiment, the antibody formulation disclosed herein comprises polysorbate 20 at about 0.02%.

In one embodiment, the antibody formulation disclosed herein comprises: (1) a histidine buffer at about 20 mM; (2) a stabilizer of arginine or a salt thereof at about 50 mM to about 200 mM; (3) an anti-PCSK9 antibody or an antigen-binding fragment thereof at about 100 mg/mL to about 200 mg/mL; and (4) polysorbate 20 at about 0.02%, wherein the antibody or the antigen-binding fragment thereof comprises HCDR1, HCDR2, HCDR3, LCDR1, LCDR2 and LCDR3, wherein, HCDR1 has an amino acid sequence of SEQ ID NO: 1; HCDR2 has an amino acid sequence of SEQ ID NO: 2; HCDR3 has an amino acid sequence of SEQ ID NO: 3; LCDR1 has an amino acid sequence of SEQ ID NO: 4; LCDR2 has an amino acid sequence of SEQ ID NO: 5; and LCDR3 has the amino acid sequence SEQ ID NO: 6.

In one embodiment, the antibody formulation disclosed herein has at least 94% of the antibody in its native conformation after 28 days of storage at 40 °C.

In one embodiment, the antibody formulation disclosed herein has at least 45% of the antibody in its main charged variants after 28 days of storage at 40 °C.

In one embodiment, the antibody formulation disclosed herein has at least 98% of the antibody in its native conformation after 12 months of storage at 2-8 °C.

In one embodiment, the antibody formulation disclosed herein has at least 87% of the antibody in its main charged variants after 12 months of storage at 2-8 °C.

In one aspect, the present invention provides a delivery device, comprising any of the antibody formulations disclosed herein.

In one aspect, the present invention provides a pre-filled syringe, comprising any of the antibody formulations disclosed herein.

In one aspect, the present invention provides use of the delivery device or the pre-filled syringe comprising any of the antibody formulations disclosed herein in treating, preventing or improving any diseases or disorders associated with PCSK9 activity.

### BRIEF DESCRIPTION OF THE DRAWINGS

FIG. 1 illustrates a JS002containing formulation inhibiting the binding and uptake of LDL by cells.
FIG. 2 illustrates a 78-day observation of LDL-C after multiple subcutaneous JS002 injections in rhesus monkeys with hyperlipidaemia.

### DETAILED DESCRIPTION

The present invention provides a stable aqueous pharmaceutical formulation comprising an anti-PCSK9 antibody or an antigen-binding portion thereof, and having improved properties compared to recognized formulations in the art. The highly concentrated formulation disclosed herein features unexpected characteristics, i.e., high stability and low viscosity.

It should be understood that the present invention is not limited to particular methods, agents, compounds, compositions or biological systems, which can, of course, be modified. It should also be understood that the terminology used herein is for the purpose of illustrating particular embodiments only, and is not intended to be limiting. As used in this specification and the appended claims, the singular forms "a", "an" and "the" include plural referents, unless otherwise specified. Thus, for example, reference to "a polypeptide" includes a combination of two or more polypeptides and the like.

As used herein, when referring to measurable values (e.g., amounts, durations, etc.), "about" is intended to encompass variations of ±20% or ±10% based on the particular value, including ±5%, ±1% and ±0.1%, as such variations are suitable for implementing the disclosed methods.

Unless defined otherwise, all technical and scientific terms used herein have the same meaning as commonly understood by those of ordinary skill in the art to which the present invention belongs. Although any methods and materials similar or equivalent to those described herein can be used in the practice of testing the present invention, the preferred materials and methods are described herein. In describing and claiming the present invention, the following terminology will be used.

"Therapeutically active antibody" or "therapeutic antibody" refers to an antibody for therapeutic purposes, i.e., for treating a disorder in a subject. It should be noted that although therapeutic proteins may be used for therapeutic purposes, the present invention is not limited to such use, as such proteins may also be used for *in vitro* studies.

The term "pharmaceutical formulation" or "formulation" refers to an product in a form such that the biological activity of the active ingredient remains effective and free of other ingredients with unacceptable toxicity to the subject to which the formulation is administered. The formulation is sterile.

The term "liquid formulation" refers to a formulation in liquid state and is not intended to refer to a resuspended lyophilized formulation. The liquid formulations of the present invention are stable during storage and their stability is independent of lyophilization (or other state change methods, such as spray drying).

The term "aqueous formulation" refers to a liquid formulation using water as the solvent. In one embodiment, the aqueous formulation is one that requires no lyophilization, spray drying, and/or freezing to maintain its stability (e.g., chemical and/or physical stability and/or biological activity).

The term "excipient" refers to an agent that may be added to a formulation to provide a desired property (e.g., consistency, improved stability) and/or to adjust osmolality. Examples of commonly used excipients include, but are not limited to, sugars, polyols, amino acids, surfactants and polymers.

As used herein, the term "buffer providing a pH of about 5.5 to about 6.5" refers to such an agent that, through the action of its acid/base conjugate components, renders a solution containing the agent resistant to pH changes. The buffer used in the formulations of the present invention may have a pH in the range of about 5.5 to about 6.5, or a pH in the range of about 5.5 to about 6.0. In one embodiment, the pH is about 6.0.

Examples of "buffers" controlling pH within this range described herein include acetate (e.g., sodium acetate), succinate (e.g., sodium succinate), gluconic acid, histidine and/or salts thereof, methionine, citrate, phosphate, citrate/phosphate, imidazole and a combination thereof, and other organic acid buffers. In one embodiment, the buffer is not a protein. In one embodiment, the buffer is histidine and/or a salt thereof, preferably L-histidine and/or a salt thereof. Generally, the concentration of the buffer in the formulation may be in the range of 5-100 mM, or is about 5 mM, 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 95 mM or 100 mM or in a range with any two of the values as endpoints, such as 10-30 mM or 15-25 mM. In one embodiment, the concentration of the buffer is about 20 mM.

"Histidine buffer" is a buffer comprising histidine and/or a salt thereof. Salts of histidine include one or more of histidine hydrochloride, histidine acetate, histidine phosphate and histidine sulfate. In one embodiment of the present invention, the histidine buffer is: a histidine buffer prepared by 1-10 mM L-histidine and 10-20 mM L-histidine monohydrochloride. In one embodiment, the histidine formulation is: a histidine buffer, pH 6.0, prepared by 4.5 mM L-histidine and 15.5 mM L-histidine monohydrochloride. In one embodiment, the histidine formulation is: a histidine buffer, pH 5.5, prepared by 9.5 mM L-histidine and 10.5 mM L-histidine monohydrochloride. In some embodiments, the histidine buffer consists of histidine and histidine hydrochloride in a molar ratio of 1:1-1:4.

As used herein, the term "surfactant" generally includes agents that protect proteins, such as antibodies, from gas/liquid interface-induced stress, liquid/solid-induced stress to reduce aggregation of the antibodies or minimize the formation of particulate matters in the formulation. Exemplary surfactants include, but are not limited to, nonionic surfactants such as polyoxyethylene sorbitan fatty acid esters (e.g., polysorbate 20 and polysorbate 80), polyethylene-polypropylene copolymers, polyethylene-polypropylene glycol, polyoxyethylene stearate, polyoxyethylene alkyl ethers (e.g., polyoxyethylene monolauryl ether), alkylphenyl polyoxyethylene ether (Triton-X), polyoxyethylene-polyoxypropylene copolymers (poloxamers, Pluronics), and sodium dodecyl sulfate (SDS). In one embodiment, the nonionic surfactant is polysorbate 20. In one embodiment, the concentration of polysorbate 20 is about 0 to 0.1% (w/v). In one embodiment, the concentration of polysorbate 20 is about 0.02% (w/v). In one embodiment, the nonionic surfactant is polysorbate 80. In one embodiment, the concentration of polysorbate 80 is about 0 to 0.1% (w/v). In one embodiment, the concentration of polysorbate 80 is about 0.02% (w/v).

As used herein, the term "stabilizer" refers to an agent that reduces aggregation of antibodies and other proteins. Exemplary stabilizers include, but are not limited to: human serum albumin (HSA), bovine serum albumin (BSA), α-casein, globin, α-lactalbumin, LDH, lysozyme, myoglobin, ovalbumin and RNase A. Stabilizers also include amino acids and metabolites thereof and salts thereof such as hydrochlorides, for example, arginine, glycine, alanine (α-alanine, β-alanine), betaine, leucine, lysine, glutamic acid, aspartic acid, proline, 4-hydroxyproline, sarcosine, γ-aminobutyric acid (GABA), opines (alanopine, octopine, strombine), and trimethylamine N-oxide (TMAO). Stabilizers also include sugars, polyols, and their metabolites, such as NaCl, KC1, MgCl₂, CaCl₂, sucrose, mannitol, and sorbitol. In one embodiment, the stabilizer is mannitol. In one embodiment, the stabilizer is sucrose. In one embodiment, the stabilizer is sorbitol. In one embodiment, the stabilizer is an amino acid or a salt thereof. In one embodiment, the amino acid is arginine or arginine hydrochloride. In one embodiment, the concentration of arginine or arginine hydrochloride is about 20-200 mM. In one embodiment, the concentration of arginine or arginine hydrochloride is about 50-200 mM. In one embodiment, the concentration of arginine or arginine hydrochloride is about 60 mM, 130 mM, 160 mM, or 165 mM.

The term "viscosity" as used herein may be "kinematic viscosity" or "absolute viscosity". "Kinematic viscosity" is a measure of the resistive flow of a fluid generated under the influence of gravity. "Absolute viscosity", sometimes referred to as dynamic viscosity or simple viscosity, is the product of kinematic viscosity and fluid density (absolute viscosity = kinematic viscosity × density). The kinematic viscosity is denoted by L²/T, where L is length and T is time. Generally, the kinematic viscosity is expressed in centistoke (cSt). The SI unit for kinematic viscosity is mm²/s, i.e., 1 cSt. The absolute viscosity is expressed in centipoise (cP). The SI unit for absolute viscosity is Milli t. s (mPa·s), wherein 1 cP = 1 mPa·s.

For the liquid formulations disclosed herein, the term "low viscosity" as used herein shall refer to an absolute viscosity less than about 15 centipoise (cP). For example, the liquid formulation disclosed herein will be considered having a "low viscosity" if the formulation exhibits an absolute viscosity of about 15 cP, about 14 cP, about 13 cP, about 12 cP, about 11 cP, about 10 cP, about 9 cP, about 8 cP or less as measured by standard viscosity measurement techniques. For the liquid formulations disclosed herein, the term "moderate viscosity" as used herein shall refer to an absolute viscosity between about 35 cP and about 15 cP. For example, the liquid formulation disclosed herein will be considered having a "moderate viscosity" if the formalation exhibits an absolute viscosity of about 34 cP, about 33 cP, about 32 cP, about 31 cP, about 30 cP, about 29 cP, about 28 cP, about 27 cP, about 26 cP, about 25 cP, about 24 cP, about 23 cP, about 22 cP, about 21 cP, about 20 cP, about 19 cP, about 18 cP, about 17 cP, about 16 cP or about 15 cP as measured by standard viscosity measurement techniques. The liquid pharmaceutical formulations disclosed herein may exhibit low to moderate viscosity in some embodiments. In one embodiment, the inventor surprisingly discovered that a liquid formulation with low viscosity is prepared by the antibody at about 100-200 mM and arginine or a salt thereof at 60-165 mM. In one embodiment, it is further discovered that arginine or a salt thereof can significantly reduce the viscosity of a formulation containing other tonicity agents such as sucrose, sorbitol, mannitol, and the like.

"Isotonic" refers to a formulation having substantially equivalent osmotic pressure to human blood. An isotonic formulation generally has an osmotic pressure of about 250 to 350 mOsm. Isotonicity can be measured by a vapor pressure osmometer or cryoscopic osmometer.

A "stable" formulation is one in which the antibody substantially retains its physical and/or chemical stability and/or biological activity during the manufacturing process and/or upon storage. A pharmaceutical formulation can be stable even if the contained antibody fails to retain 100% of its chemical structure or biological function after a certain period of storage. In certain instances, a pharmaceutical formulation that retains about 90%, about 95%, about 96%, about 97%, about 98%, or about 99% of antibody structure or function after a certain period of storage may also be considered "stable". Various analytical techniques for measuring protein stability are available in the art and are described in Peptide and Protein Drug Delivery, 247-301, Vincent Lee ed., Marcel Dekker, Inc., New York, N.Y., Pubs. (1991) and Jones, A., (1993) Adv. Drug Delivery Rev., 10:29-90, both of which are incorporated herein for reference.

After a certain period of storage at a certain temperature, the stability of the formulation can be measured by determining the percentage of remaining native antibody (among other methods). Except other methods, the percentage of native antibody can be measured by size-exclusion chromatography (e.g., size-exclusion high-performance liquid chromatography [SE-HPLC]), "native" refers to unaggregated and undegraded. In some embodiments, the stability of a protein is determined by a percentage of monomeric protein in a solution having a low percentage of degraded (e.g., fragmented) and/or aggregated protein. In one embodiment, the formulation is stable at room temperature, about 25-30 °C, or 40 °C for at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months or longer, having no more than about 6%, 5%, 4%, 3%, 2%, 1%, 0.5%, or 0.1% of antibody in aggregated form.

Stability can be measured by determining the percentage of migrating antibody in a fraction that is more acidic ("acidic form") than the main fraction of the antibody ("main charged form") during ion exchange (and can be measured by other methods), wherein the stability is inversely proportional to the percentage of acidic form of antibody. The percentage of "acidified" antibody can be measured by, except other methods, ion exchange chromatography (e.g., cation exchange high-performance liquid chromatography [CEX-HPLC]). In one embodiment, an acceptable stability means that the acidic form of the antibodies that can be detected in the formulation is no more than about 49%, 45%, 40%, 35%, 30%, 25%, 20%, 15%, 10%, 5%, 4%, 3%, 2%, 1%, 0.5% or 0.1% formulation after a certain period of storage at a certain temperature. The certain period of storage prior to measuring stability can be at least 2 weeks, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or longer. When assessing the stability, the certain temperature that allows the storage of the pharmaceutical formulation can be any temperature in the range of about -80 °C to about 45 °C, e.g., about -80 °C, about -30 °C, about -20 °C, about 0 °C, about 2-8 °C, about 5 °C, about 25 °C, or about 40 °C.

An antibody in pharmaceutical formulation "retains its physical stability" if the antibody does not substantially show signs of, such as aggregation, precipitation, and/or denaturation, during visual inspection of color and/or clarity or by UV light scattering or measurement by pore exclusion chromatography. Aggregation is a process in which individual molecules or complexes associate, covalently or non-covalently, to form aggregates. Aggregation can proceed to the point where visible precipitates are formed. Stability, e.g., physical stability, of a formulation can be assessed by methods well known in the art, including measuring the apparent extinction (absorbance or optical density) of a sample. Such extinction measurement correlates with the turbidity of the formulation. Turbidity of a formulation is, in part, an inherent property of proteins dissolved in solution and is generally measured by nephelometry and is measured in nephelometric turbidity unit (NTU).

Turbidity levels that vary with, for example, the concentration of one or more components in a solution (e.g., protein and/or salt concentration) are also referred to "opacification" or "opacified appearance" of a formulation. Turbidity levels can be calculated with reference to a standard curve based on suspensions of known turbidity. The reference standards for determining the turbidity level of a pharmaceutical composition may be based on the standard of the European Pharmacopoeia, 4th edition, Directorate for the Quality of Medicine of the Council of Europe (EDQM), Strasbourg, France. According to the European Pharmacopoeia standard, a clear solution is defined as a solution having a turbidity lower than or equal to that of a reference suspension according to the European Pharmacopoeia standard having a turbidity of about 3. Turbidity measurement by nephelometry can measure Rayleigh scattering in the absence of association or non-ideal effects, which generally varies linearly with concentration. Other methods for assessing physical stability are well known in the art.

An antibody "retains its chemical stability" in a pharmaceutical formulation if the chemical stability of the antibody at a given time point allows the antibody to retainits biological activity as defined hereinafter. Chemical stability can be assessed, for example, by detecting or quantifying the form of chemical changes in the antibody. Chemical changes can include size changes (e.g., truncation), which can be assessed by, for example, size-exclusion chromatography, SDS-PAGE, and/or matrix-assisted laser desorption/ionisation time-of-flight mass spectrometry (MALDI/TOF MS). Other types of chemical changes include charge changes (e.g., occurring as a result of deamidation or oxidation), which can be assessed by, for example, ion exchange chromatography. An antibody in a pharmaceutical formulation "retains its biological activity" if it is biologically active for its intended purpose. For example, a formulation described herein is considered stable if, after a certain period of storage (e.g., 1 to 12 months) at a certain temperature, for example, 5 °C, 25 °C or 45 °C, the binding affinity of the anti-PCSK9 antibody in the formulation for PCSK9 is at least 90%, 95% or greater of that of the antibody prior to storage. Binding affinity can also be determined by, for example, ELISA or plasmon resonance.

In the context of the present invention, a "therapeutically effective amount" or "effective amount" of an antibody, pharmacologically refers to an amount that is effective in preventing, treating or alleviating the symptoms of a disorder that the antibody can effectively treat.

The term "subject" or "patient" is intended to include mammalian organisms. Examples of subjects/patients include human and non-human mammals such as non-human primates, dogs, cows, horses, pigs, sheep, goats, cats, mice, rabbits, rats and transgenic non-human animals. In one specific embodiment of the present invention, the subject is human.

### Anti-PCSK9 antibody

The formulations disclosed herein comprise an antibody or an antigen-binding fragment thereof that specifically binds to human PCSK9. As used herein, the term "PCSK9" is a human proprotein convertase, which belongs to the proteinase K subfamily of the subtilisin family. Literatures have demonstrated that PCSK9 can increase plasma LDL level by binding to low-density lipoprotein receptor and promoting its degradation. The term "antibody" as used herein should be construed including intact antibody molecules and antigen-binding fragments thereof. The term "antigen-binding moiety" or "antigen-binding fragment" of an antibody (or simply "antibody moiety" or "antibody fragment"), as used herein, refers to one or more fragments of an antibody that retain the ability to specifically bind to human PCSK9 or an epitope thereof.

The term "full-length antibody", as used herein, refers to an immunoglobulin molecule comprising four peptide chains, including two heavy (H) chains (about 50-70 kDa in total length) and two light (L) chains (about 25 kDa in total length) linked to each other by disulfide bonds. Each heavy chain consists of a heavy chain variable region (abbreviated herein as VH) and a heavy chain constant region (abbreviated herein as CH). The heavy chain constant region consists of 3 domains CHI, CH2 and CH3. Each light chain consists of a light chain variable region (abbreviated herein as VL) and a light chain constant region. The light chain constant region consists of one domain CL. The VH and VL regions can be further divided into complementarity determining regions (CDRs) with high variability and more conservative regions called framework regions (FRs) that are spaced apart by the CDRs. Each VH or VL region consists of 3 CDRs and 4 FRs, in the following order from the amino terminus to the carboxy terminus: FR1, CDR1, FR2, CDR2, FR3, CDR3, and FR4. The variable regions of the heavy and light chains contain binding domains that interact with antigens. The constant region of the antibody can mediate the binding of immunoglobulins to host tissues or factors, including the binding of various cells of the immune system (e.g., effector cells) to the first component (C1q) of classical complement system.

As used herein, the term "CDR" refers to the complementarity determining regions within an antibody variable sequence. There are 3 CDRs in each variable region of the heavy and light chains, which are named HCDR1, HCDR2 and HCDR3, and LCDR1, LCDR2 and LCDR3 for heavy and light chain variable regions, respectively. Exact boundaries of the CDRs may vary among different systems. The aforementioned system described by Kabat provides not only a clear residue numbering system applicable to any variable region of an antibody, but also exact residue boundaries defining the 3 CDRs. Such CDRs may be referred to as Kabat CDRs. Chothia et al. found that certain subsections within the Kabat CDRs demonstrate almost identical peptide backbone configurations despite the significant diversity in amino acid sequence (Chothia et al., (1987) Mol. Biol. 196:901-917; Chothia et al., (1989) Nature 342:877-883). Other boundaries defining CDRs which overlap with the Kabat CDRs have been described by Padlan, (1995) FASEB J. 9:133-139 and MacCallum, (1996) J. Mol. Biol. 262(5):732-45. Still other CDR boundary definitions may not strictly follow one of the systems described herein. However, although such CDRs may be shortened or lengthened, they still overlap with the Kabat CDRs, since a particular residue or set of residues, or even the entire CDR, does not significantly affect the antigen binding according to prediction or experimental findings. The methods disclosed herein may utilize CDRs defined by any of these systems, although certain embodiments use CDRs defined by Kabat or Chothia.

The anti-PCSK9 antibodies or antigen-binding fragments thereof described herein include any of the anti-PCSK9 antibodies described in International Patent Publication No. WO2017088782. In one embodiment, the antibody used in the methods and compositions disclosed herein comprises the 6 CDRs from antibody JS002.

"Antigen-binding fragment" as used herein includes a fragment or derivative of an antibody, generally including at least one fragment of an antigen-binding region or variable region (e.g., one or more CDRs) of a parent antibody, which retains at least some of the binding specificity of the parent antibody. Examples of antigen-binding fragments include, but are not limited to, Fab, Fab', F(ab')₂ and Fv fragments; a diabody; a linear antibody; a single-chain antibody molecule, such as sc-Fv; a nanobody and multispecific antibody formed by antibody fragments. A binding fragment or a derivative generally retains at least 10% of its antigen binding activity when the antigen binding activity is presented on a molar concentration basis. Preferably, the binding fragment or derivative retains at least 20%, 50%, 70%, 80%, 90%, 95% or 100% or more of the antigen binding affinity of the parent antibody. It is also contemplated that an antigen-binding fragment of an antibody may include conservative or non-conservative amino acid substitutions that do not significantly alter their biological activity (referred to as a "conservative variant" or "function-conservative variants" of the antibody).

In one embodiment, the anti-PCSK9 antibody or the antigen-binding fragment thereof described herein comprises HCDR1, HCDR2, HCDR3, LCDR1, LCDR2 and LCDR3, wherein, HCDR1 has an amino acid sequence of SEQ ID NO: 1; HCDR2 has an amino acid sequence of SEQ ID NO: 2; HCDR3 has an amino acid sequence of SEQ ID NO: 3; LCDR1 has an amino acid sequence of SEQ ID NO: 4; LCDR2 has an amino acid sequence of SEQ ID NO: 5; and LCDR3 has the amino acid sequence SEQ ID NO: 6. In one embodiment, the anti-PCSK9 antibody or the antigen-binding fragment thereof described herein comprises a heavy chain variable region (VH) having an amino acid sequence of SEQ ID NO: 7 and a light chain variable region (VL) having an amino acid sequence of SEQ ID NO: 8.

A non-limiting, exemplary antibody used in the examples herein is referred to as "JS002", which is a fully humanized antibody that specifically binds to human PCSK9, comprising a heavy chain having an amino acid sequence of SEQ ID NO: 9 and a light chain having an amino acid sequence of SEQ ID NO: 10.

### Pharmaceutical formulation

The formulation disclosed herein is a liquid formulation comprising a highly concentrated active antibody and having high stability and low viscosity. In particular, the present invention found that a formulation comprising an arginine salt has a significantly lower viscosity than a formulation comprising a tonicity agent. In addition, adding an arginine salt in the formulation buffer can significantly reduce the viscosity as compared with a formulation buffer comprising a tonicity agent.

The formulation disclosed herein comprises an antibody or an antigen-binding fragment thereof that specifically binds to human PCSK9, a buffer, and a stabilizer. Preferably, the pH of the formulation disclosed herein is in the range of 5.5 to 6.5.

In the formulation disclosed herein, the concentration of the anti-PCSK9 antibody or the antigen-binding fragment thereof generally ranges from 100 mg/mL to about 200 mg/mL. In some embodiments, the formulation disclosed herein comprises 150±10 mg/mL of the anti-PCSK9 antibody or the antigen-binding fragment thereof. In one preferred embodiment, the anti-PCSK9 antibody or the antigen-binding fragment thereof described herein comprises HCDR1, HCDR2, HCDR3, LCDR1, LCDR2 and LCDR3, wherein, HCDR1 has an amino acid sequence of SEQ ID NO: 1; HCDR2 has an amino acid sequence of SEQ ID NO: 2; HCDR3 has an amino acid sequence of SEQ ID NO: 3; LCDR1 has an amino acid sequence of SEQ ID NO: 4; LCDR2 has an amino acid sequence of SEQ ID NO: 5; and LCDR3 has the amino acid sequence SEQ ID NO: 6. More preferably, the antibody or the antigen-binding fragment thereof comprises a heavy chain variable region (VH) having an amino acid sequence of SEQ ID NO: 7 and a light chain variable region (VL) having an amino acid sequence of SEQ ID NO: 8. More preferably, the antibody or the antigen-binding fragment thereof comprises a heavy chain (HC) having an amino acid sequence of SEQ ID NO: 9 and a light chain (LC) having an amino acid sequence of SEQ ID NO: 10.

In the formulation disclosed herein, the buffer may be an acetate buffer or a histidine buffer, preferably a histidine buffer. Preferably, the pH of the buffer is in the range of 5.5-6.0. Preferably, the histidine buffer disclosed herein comprises histidine and a salt of histidine. Generally, the histidine buffer described herein comprises 1-10 mM of histidine and 10-20 mM of a histidine salt (e.g., monohydrochloride). Preferably, the molar ratio of histidine to the salt of histidine in the histidine buffer is in the range of 1:1 to 1:4. In the formulation disclosed herein, the buffer can be present at a concentration of 10-50 mM, preferably 15-25 mM, e.g., 20 mM.

In the formulation disclosed herein, the stabilizer may be selected from one or more of arginine or a salt thereof, sorbitol, mannitol and sucrose, preferably an arginine salt. Exemplary arginine salts include arginine hydrochloride. Other arginine salts pharmaceutically suitable as a stabilizer may also be used in the present invention. In general, the concentration of the stabilizer in the formulation disclosed herein can be in the range of 50 mM to 300 mM, for example, 50-200 mM, 130-200 mM or 160-250 mM. When the arginine salt is used alone, its concentration in the formulation can be in the range of 50-200 mM, such as 130-200 mM. In some embodiments, the stabilizer is a mixture of an arginine salt and mannitol or sorbitol. When the arginine salt is used in combination with mannitol or sorbitol, the concentration of the arginine salt in the formulation can be in the range of 50-150 mM, and mannitol or sorbitol in the range of 50-200 mM. The total concentration of the two stabilizers can be in the range of 50 mM to 300 mM, e.g., 50 mM to 250 mM.

In some embodiments, the formulation disclosed herein comprise: (1) an anti-PCSK9 antibody or antigen-binding fragment thereof; (2) a histidine buffer at about pH 5.5-6.5; and (3) an arginine salt.

In some embodiments of the present invention, the formulation disclosed herein further comprise a nonionic surfactant as described herein. Generally, when present, the concentration of the nonionic surfactant in the formulation is not more than 0.1% (w/v), such as 0.01-0.05% (w/v). Preferred nonionic surfactants include polysorbate 20 and/or polysorbate 80, with a particularly preferred concentration of about 0.02%.

In one embodiment, the formulation disclosed herein comprises: (1) the anti-PCSK9 antibody or the antigen-binding fragment thereof at about 100 mg/mL to about 200 mg/mL; (2) a histidine buffer at about 10-50 mM, about pH 5.5-6.5; (3) an arginine salt at about 50 mM to about 200 mM; and (4) a nonionic surfactant at about 0% to about 0.1%.

In one embodiment, the formulation disclosed herein comprises: (1) the anti-PCSK9 antibody or the antigen-binding fragment thereof at about 150±10 mg/mL; (2) a histidine buffer at about 20 mM, about pH 5.5-6.5; (3) an arginine salt at about 60±5 mM, 130±5 mM or 160±5 mM; and (4) a nonionic surfactant at about 0% to about 0.1%.

In one embodiment, the formulation disclosed herein comprises: (1) the anti-PCSK9 antibody or the antigen-binding fragment thereof at about 150±10 mg/mL; (2) a histidine buffer at about 20 mM, about pH 5.5-6.5; (3) an arginine salt at about 160±5 mM; and (4) polysorbate 20 at about 0.02%.

In one embodiment, the formulation disclosed herein comprises: (1) the anti-PCSK9 antibody or the antigen-binding fragment thereof at about 150±10 mg/mL, wherein the antibody comprises an HCDR1 having an amino acid sequence of SEQ ID NO: 1, an HCDR2 having an amino acid sequence of SEQ ID NO: 2, an HCDR3 having an amino acid sequence of SEQ ID NO: 3, an LCDR1 having an amino acid sequence of SEQ ID NO: 4, an LCDR2 having an amino acid sequence of SEQ ID NO: 5, and an LCDR3 having the amino acid sequence SEQ ID NO: 6; (2) a histidine buffer at about 20 mM, about pH 5.5-6.5; (3) arginine at about 160±5 mM; and (4) polysorbate 20 at about 0.02%.

In one embodiment, the formulation disclosed herein has a pH of 5.5-6.5 and comprises: (1) the anti-PCSK9 antibody or the antigen-binding fragment thereof at about 150±10 mg/mL, wherein the antibody comprises a heavy chain variable region (VH) having an amino acid sequence of SEQ ID NO: 7 and a light chain variable region (VL) having an amino acid sequence of SEQ ID NO: 8; (2) a histidine buffer at about 20 mM, about pH 5.5-6.5; (3) arginine at about 160±5 mM; and (4) polysorbate 20 at about 0.02%.

In one embodiment, the formulation disclosed herein comprises: (1) the anti-PCSK9 antibody or the antigen-binding fragment thereof at about 150±10 mg/mL, wherein the antibody is a full-length antibody comprising a heavy chain having an amino acid sequence of SEQ ID NO: 9 and a light chain having an amino acid sequence of SEQ ID NO: 10; (2) a histidine buffer at about 20 mM, about pH 5.5-6.5; (3) arginine at about 160±5 mM; and (4) polysorbate 20 at about 0.02%.

### Containers for the pharmaceutical formulation and routes of administration

The pharmaceutical formulation disclosed herein may be contained in any container suitable for storing pharmaceuticals and other pharmaceutical compositions. For example, the pharmaceutical formulation disclosed herein may be contained in a sealed and sterilized plastic or glass container, such as a vial, ampoule, syringe, cylinder or bottle, of a certain volume. Different types of vials can be used to contain the formulations of the present invention, including, for example, transparent and opaque (e.g., brown) glass or plastic vials. Likewise, different types of syringes may be employed to contain and/or administer the pharmaceutical formulation disclosed herein. The pharmaceutical formulation can be administered to a patient via parenteral routes, such as injection (e.g., subcutaneous, intravenous, intramuscular, intraperitoneal, etc.), or transdermal, mucosal, nasal, respiratory, and/or buccal administration. A variety of reusable pens and/or autoinjectors may be employed to subcutaneously deliver the pharmaceutical formulation disclosed herein. Examples of disposable pens and/or autoinjectors that may be used for subcutaneous delivery of the pharmaceutical composition disclosed herein include, but are not limited to, SOLOSTAR™ pens (Sanofi-Aventis), FLEXPEN™ (Novo Nordisk), KWIKPEN™ (Eli Lilly), SURECLICK™ autoinjector (Amgen, Thousand Oaks, CA), PENLET™ (Haselmeier, Stuttgart, Germany), EPIPEN (Dey, L. P.) and HUMIRA™ pens (Abbott Labs, Abbott Park, IL), and many others.

Use of a micro-infusion set for delivering the formulation disclosed herein is also included. The term "micro-infusion set" as used herein refers to a subcutaneous delivery device designed for slow administration of a large volume (e.g., about 2.5 mL or more) of a therapeutic formulation over a long period of time (e.g., about 10, 15, 20, 25, 30 minutes or longer). See, e.g., U.S. Pat. Nos. 6,629,949 and 6,659,982, and Meehan et al., J. Controlled Release 46:107-116 (1996). Micro-infusion sets are particularly suitable for delivering large doses of therapeutic proteins in solutions of high concentration (e.g., about 100, 125, 150, 175, 200 mg/mL or higher) and/or viscosity.

### Therapeutic use of pharmaceutical formulations

Among other uses, the pharmaceutical formulation disclosed can be used in treating, preventing, or improving any disease or disorder associated with PCSK9 activity, including PCSK9-mediated diseases or disorders. Exemplary, non-limiting diseases and disorders that may be treated or prevented by the pharmaceutical formulations disclosed herein include various dyslipidemias, such as hypercholesterolemia, familial hypercholesterolemia, 1¾ lipidemia, familial 1¾ lipidemia, dyslipoproteinemia type 3, familial dyslipoproteinemia type 3, 1¾ hypertriglyceridemia and familial hypertriglyceridemia.

The present invention also provides use of the antibody formulation disclosed herein in preparing a medicament for treating, preventing or improving any of the diseases or disorders associated with PCSK9 activity described herein. Also provided are antibody formulations described herein for treating, preventing, or improving any of the diseases or disorders associated with PCSK9 activity described herein.

The present invention is described in detail below by way of examples, which, however, are not intended to limit the present invention. The present invention has been described in detail and specific embodiments are disclosed. It will be apparent for those skilled in the art that various changes and modifications can be made to the specifis examples without departing from the spirit and scope of the present invention.

### Example 1. Buffer system and pH selection

In a liquid antibody formulation, the buffer system and pH closely affect the stability of the antibody, and each antibody having unique physicochemical properties may have an optimum buffer and pH. To select an optimal buffer system and pH will provide the anti-PCSK9 antibody disclosed herein with optimal stability for clinical application. The study was conducted with JS002 at about 150 mg/mL, with 130 mM arginine hydrochloride being the excipient. The buffer of JS002 was changed into corresponding buffers by dialysis tubing, and the samples were sealed in a centrifuge tube for testing. Both sodium acetate buffer and histidine buffer (the molar ratio of histidine to histidine hydrochloride was 1:1) at pH 5.0 to 6.0 were subjected to the testing (as shown in Table 1). The samples were incubated at 40 °C/ambient RH and analyzed at weeks 2 and 4. The major degradation pathways of the protein were the formations of aggregates, cleavage products and charged variants. The percentages of native (monomeric) and aggregated JS002 were determined by size exclusion chromatography (SEC-HPLC) and the percentages of acidic and basic mAb were determined by cation exchange chromatography (CEX-HPLC). The influence of different buffer systems and pH on the JS002 antibody stability was examined at baseline (0 w), two weeks (2 w) and four weeks (4 w). By linear fitting, straight lines were plotted according to the SEC-HPLC monomer content and the CEX-HPLC main peak content and the slopes (%/week) was calculated. The results are summarized in Table 2.

**Table 1. Formulations for buffer system and pH selection**

| Formulation no. | pH | Buffer system | Excipient |
|---|---|---|---|
| 1 | 5.0 | 20 mM sodium acetate buffer | 130 mM arginine hydrochloride |
| 2 | 5.5 | 20 mM sodium acetate buffer | 130 mM arginine hydrochloride |
| 3 | 6.0 | 20 mM histidine buffer | 130 mM arginine hydrochloride |

**Table 2. Summary of stability in buffer systems and pH selection**

| Formulation no. | Temperature Time | SEC-HPLC Monomer (%) | SEC-HPLC Monomer decline (%/week) | CEX-HPLC Main peak (%) | CEX-HPLC Main peak decline (%/week) |
|---|---|---|---|---|---|
| 1 | 0 week | 99.4 | 1.20 | 86.4 | 5.90 |
| | 40 °C, 2 weeks | 98.5 | | 75.8 | |
| | 40 °C, 4 weeks | 94.6 | | 62.8 | |
| 2 | 0 week | 99.3 | 0.82 | 86.2 | 8.48 |
| | 40 °C, 2 weeks | 98.8 | | 75.4 | |
| | 40 °C, 4 weeks | 96.0 | | 52.3 | |
| 3 | 0 week | 99.5 | 0.57 | 86.5 | 9.75 |
| | 40 °C, 2 weeks | 99.0 | | 77.9 | |
| | 40 °C, 4 weeks | 97.2 | | 47.5 | |

As shown in Table 2, in SEC-HPLC analysis, the antibody was relatively stable at pH 5.5-6.0. After a 4-week storage at 40 °C, the monomer content in the samples was over 94%, and the monomer content decline was less than 1.5%/week; the main charged form in the samples consisted of more than 45%, and the decline of the main charged form was less than 10%/week. For histidine buffer, pH 6.0 (formulation no. 3), the monomer content was highest (approximately 97%) after 4 weeks at 40 °C, and the decline in monomer content was only 0.57%/week. Based on these results, 20 mM histidine buffer, pH 5.5-6.0, was selected for the formulation of liquid JS002 formulation.

### Example 2. Stabilizer selection

To further explore the effect of different excipients on antibody stability and viscosity, formulations comprising one or a combination of sucrose, arginine hydrochloride, sorbitol or mannitol were selected for comparative testing. The aforementioned excipients or combinations thereof were added to 20 mM histidine buffer (pH 5.5 or 6.0) containing JS002 at about 150 mg/mL. The specific formulations are shown in Table 3. The formulations were separately packaged and incubated at 40 °C, and were subjected to testings at 2 weeks and 4 weeks for antibody stability and formulation viscosity. Changes in JS002 monomer content was determined by size-exclusion high-performance liquid chromatography (SEC-HPLC), the charged main peak content by cation exchange high-performance liquid chromatography (CEX-HPLC) and the viscosity of the formulation at four weeks by a standard method. As shown in Table 4, in a formulation comprising one or a combination of sucrose, arginine hydrochloride, sorbitol or mannitol, the antibody exhibited strong thermal stability. After a 4-week storage at 40 °C, the content of the sample monomer was over 98%, and the main charged form consisted of more than 51%.

Particularly, the formulation comprising the arginine salt alone exhibited the highest stability and the lowest viscosity. Specifically, after a 4-week storage at 40 °C, the formulation comprising the arginine salt alone demonstrated: (1) for antibody structural stability, the lowest decline in antibody monomer content down to 0.1%/week which was about 25-30% of that of the highest formulation (sucrose alone), the highest antibody monomer content up to 98.8%, and a certain improvement in enhancement of sucrose, sorbitol or mannitol on antibody stability; (2) for stability of, for example, charged forms, the lowest decline in main charged antibody form down to 6.25%/week, with a main charged form content up to 61.2%; and (3) a viscosity below 5 cP (Table 5), which was significantly lower than the other formulations, especially about 20-25% of that of sucrose alone. In addition, the arginine salt significantly improved the viscosity of sucrose, sorbitol or mannitol-containing formulations by a reduction in viscosity of about 50%.

Highly concentrated antibody solutions are generally more prone to aggregation, precipitation, etc., resulting in reduced antibody stability, and increased solution viscosity may lead to difficulty in injection (particularly subcutaneous or intramuscular injection). In summary, the arginine salt in the liquid formulation can ensure the stability of the antibody JS002 and significantly reduce the viscosity. Particular, for histidine buffer (pH 5.5 or 6.0), adding an arginine salt alone as stabilizer provides the optimal antibody stability and formulation viscosity.

**Table 3. Formulations for stabilizer selection**

| Formulation no. | pH | Excipient |
|---|---|---|
| 4 | 5.5 | 240 mM sucrose |
| 5 | 6.0 | 240 mM sucrose |
| 6 | 5.5 | 165 mM arginine hydrochloride |
| 7 | 6.0 | 165 mM arginine hydrochloride |
| 8 | 5.5 | 270 mM sorbitol |
| 9 | 6.0 | 270 mM sorbitol |
| 10 | 5.5 | 260 mM mannitol |
| 11 | 6.0 | 260 mM mannitol |
| 12 | 5.5 | 60 mM arginine hydrochloride 155 mM sucrose |
| 13 | 6.0 | 60 mM arginine hydrochloride 155 mM sucrose |
| 14 | 5.5 | 60 mM arginine hydrochloride 165 mM sorbitol |
| 15 | 6.0 | 60 mM arginine hydrochloride 165 mM sorbitol |
| 16 | 5.5 | 60 mM arginine hydrochloride 160 mM mannitol |
| 17 | 6.0 | 60 mM arginine hydrochloride 160 mM mannitol |

**Table 4. Summary of stabilizer selection**

| Formulation no. | SEC-HPLC Monomer (%) | | | Monomer decline by SEC-HPLC (%/week) | CEX-HPLC Main peak (%) | | | Main peak decline by CEX-HPLC (%/week) |
|---|---|---|---|---|---|---|---|---|
| | 0 week | 2 weeks | 4 weeks | | 0 week | 2 weeks | 4 weeks | |
| 4 | 99.5 | 98.7 | 98.1 | 0.38 | 88.4 | 68.9 | 53.2 | 8.80 |
| 5 | 99.3 | 98.5 | 98.3 | 0.30 | 87.6 | 67.7 | 55.5 | 8.03 |
| 6 | 99.4 | 99.0 | 98.5 | 0.25 | 86.2 | 70.4 | 61.2 | 6.25 |
| 7 | 99.4 | 99.1 | 98.8 | 0.10 | 86.3 | 69.5 | 59.8 | 6.63 |
| 8 | 99.4 | 99.0 | 98.4 | 0.28 | 87.7 | 68.1 | 56.6 | 7.78 |
| 9 | 99.3 | 98.6 | 98.2 | 0.30 | 87.9 | 70.7 | 54.6 | 8.33 |
| 10 | 99.4 | 98.9 | 98.0 | 0.28 | 87.6 | 67.8 | 56.3 | 7.83 |
| 11 | 99.3 | 99.0 | 98.3 | 0.30 | 87.7 | 66.8 | 55.1 | 8.15 |
| 12 | 99.4 | 98.8 | 98.6 | 0.20 | 88.6 | 72.3 | 51.1 | 9.38 |
| 13 | 99.4 | 98.8 | 98.5 | 0.23 | 88.8 | 71.3 | 52.7 | 9.03 |
| 14 | 99.4 | 98.9 | 98.5 | 0.23 | 87.9 | 72.2 | 51.6 | 9.08 |
| 15 | 99.7 | 99 | 98.5 | 0.30 | 88.6 | 70 | 54 | 8.65 |
| 16 | 99.6 | 98.9 | 98.6 | 0.25 | 87.9 | 72.3 | 52.4 | 8.88 |
| 17 | 99.5 | 99 | 98.5 | 0.25 | 87.6 | 72 | 53.2 | 8.60 |

**Table 5. Viscosity of formulations**

| Formulation no. | 4 | 5 | 6 | 7 | 8 | 9 | 10 | 11 | 12 | 13 | 14 | 15 | 16 | 17 |
|---|---|---|---|---|---|---|---|---|---|---|---|---|---|---|
| Viscosity (cP) | 25.3 | 22.5 | 4.8 | 4.3 | 16.0 | 18.4 | 14.1 | 17.9 | 12.2 | 11.9 | 7.8 | 8.1 | 8.0 | 8.0 |

### Example 3. Surfactant selection

Surfactants are commonly used in liquid formulations as an agent for protecting proteins such as antibodies from gas/liquid-induced stress, liquid/solid-induced stress during storage to reduce aggregation of the antibodies or minimize the formation of particulate matter in the formulation, which facilitates the physical/chemical stability of the antibodies. Polysorbate 20 or polysorbate 80 at different concentrations (0-0.5%) was added to formulations comprising 20 mM histidine buffer (pH 6.0), 130 mM arginine hydrochloride and 150 mg/mL JS002, and the mixture was incubated at 40 °C for 2 weeks before testing. As shown in Table 6, the SEC-HPLC monomer content and CEX-HPLC main peak content measurements showed no significant difference in thermal stability of the antibody JS002 in formulations comprising different concentrations (0-0.5%) of polysorbate 20 or polysorbate 80, but all exhibited high stability.

**Table 6. Surfactant selection**

| Formulation no. | Surfactant | SEC-HPLC (%) Monomer (%) | CEX-HPLC (%) Main charged form (%) |
|---|---|---|---|
| 18 | 0.0% polysorbate 20 | 98.9 | 68.4 |
| 19 | 0.01% polysorbate 20 | 98.8 | 68.3 |
| 20 | 0.02% polysorbate 20 | 98.8 | 68.4 |
| 21 | 0.05% polysorbate 20 | 98.9 | 68.7 |
| 22 | 0.1% polysorbate 20 | 98.8 | 68.9 |
| 23 | 0.0% polysorbate 80 | 98.8 | 68.6 |
| 24 | 0.01% polysorbate 80 | 98.8 | 68.4 |
| 25 | 0.02% polysorbate 80 | 98.6 | 67.8 |
| 26 | 0.05% polysorbate 80 | 98.6 | 67.1 |
| 27 | 0.1% polysorbate 80 | 98.9 | 68.4 |

### Example 4. Long-term stability study

Liquid pharmaceutical formulations containing therapeutic antibodies generally require a temperature of 2-8 °C for storage, which is important for the stability of the formulation over a long-term storage. Based on the above selection results, 4 formulations were designed for long-term stability study.

Formulations of the 4 formulations shown in Table 7 were stored in transparent vials, and after a storage at 2-8 °C for several months, the samples were analyzed. Stability was assessed by the following parameters: (a) appearance; (b) particulate matters by light-blockage method (OD: 405 nm); (c) pH; (d) molecular weight of the antibody by CE-SDS; (d) contents of antibody monomer (specification: ≥ 97.0%), polymers (specification: ≤ 3.0%) or fragments (specification: ≤ 1.0%) by SEC-HPLC; (e) main charged form (specification: ≥ 70.0%), acidic forms (specification: ≤ 30.0%) or basic forms (specification: ≤ 15.0%) of the antibody by CEX-HPLC; (f) antibody binding activity by ELISA (specification: 70-130% of reference standard); and (g) biological activity of the antibody (LDL uptake assay in HepG2 cells; specification: 70%-130% of reference standard). As shown in Table 8, the 4 formulations exhibited very good stability during storage at 2-8 °C for 1-12 months.

**Table 7. Formulations for long-term stability study**

| Formulation no. | pH | Composition |
|---|---|---|
| 28 | 5.5 | 20 mM histidine buffer, 160 mM arginine hydrochloride, 0.02% polysorbate 20, and 150 mg/mL JS002 |
| 29 | 6.0 | 20 mM histidine buffer, 160 mM arginine hydrochloride, 0.02% polysorbate 20, and 150 mg/mL JS002 |
| 30 | 6.0 | 20 mM histidine buffer, 120 mM arginine hydrochloride, 60 nM mannitol, 0.02% polysorbate 20, and 150 mg/mL JS002 |
| 31 | 6.0 | 20 mM histidine buffer, 60 mM arginine hydrochloride, 160 nM mannitol, 0.02% polysorbate 20, and 150 mg/mL JS002 |

**Table 8. Long-term stability of formulations**

| Test item | Time (month) | Formulation | | | |
|---|---|---|---|---|---|
| | | 28 | 29 | 30 | 31 |
| Monomer (%) by SEC-HPLC | 0 | 99.7 | 99.7 | 99.7 | 99.7 |
| | 1 | 99.7 | 99.7 | 99.7 | 99.7 |
| | 2 | 99.8 | 99.8 | 99.7 | 99.7 |
| | 3 | 99.6 | 99.6 | 99.5 | 99.5 |
| | 6 | 99.5 | 99.5 | 99.5 | 99.5 |
| | 9 | 99.6 | 99.5 | 99.5 | 99.5 |
| | 12 | 99.6 | 99.6 | 99.6 | 99.6 |
| Polymer (%) by SEC-HPLC | 0 | 0.3 | 0.3 | 0.3 | 0.3 |
| | 1 | 0.3 | 0.3 | 0.3 | 0.3 |
| | 2 | 0.2 | 0.2 | 0.3 | 0.3 |
| | 3 | 0.4 | 0.4 | 0.5 | 0.5 |
| | 6 | 0.5 | 0.5 | 0.5 | 0.5 |
| | 9 | 0.4 | 0.5 | 0.5 | 0.5 |
| | 12 | 0.4 | 0.4 | 0.4 | 0.4 |
| Fragment (%) by SEC-HPLC | 0 | 0.0 | 0.0 | 0.0 | 0.0 |
| | 1 | 0.0 | 0.0 | 0.0 | 0.0 |
| | 2 | 0.0 | 0.0 | 0.0 | 0.0 |
| | 3 | 0.0 | 0.0 | 0.0 | 0.0 |
| | 6 | 0.0 | 0.0 | 0.0 | 0.0 |
| | 9 | 0.0 | 0.0 | 0.0 | 0.0 |

| Test item | Time (month) | Formulation | | | |
|---|---|---|---|---|---|
| | | 28 | 29 | 30 | 31 |
| | 12 | 0.0 | 0.0 | 0.0 | 0.0 |
| Main peak (%) by CEX-HPLC | 0 | 88.4 | 84.9 | 87.1 | 85.2 |
| | 1 | 90.3 | 89.9 | 90.6 | 88.5 |
| | 2 | 84.5 | 85.3 | 84.4 | 86.7 |
| | 3 | 88.1 | 88.2 | 88.3 | 88.1 |
| | 6 | 88.5 | 88.6 | 88.7 | 88.2 |
| | 9 | 87.8 | 84.3 | 87.7 | 88.4 |
| | 12 | 89.0 | 88.3 | 87.6 | 87.6 |
| Acidic peak (%) by CEX-HPLC | 0 | 7.8 | 9.0 | 8.0 | 8.7 |
| | 1 | 6.5 | 6.9 | 6.4 | 8.0 |
| | 2 | 8.4 | 8.8 | 10.1 | 8.1 |
| | 3 | 7.8 | 8.2 | 8.0 | 8.2 |
| | 6 | 7.9 | 8.2 | 8.2 | 8.5 |
| | 9 | 9.2 | 9.9 | 9.8 | 8.3 |
| | 12 | 8.0 | 9.1 | 9.6 | 9.5 |
| Basic peak (%) by CEX-HPLC | 0 | 3.9 | 6.1 | 4.9 | 6.1 |
| | 1 | 3.2 | 3.2 | 3.1 | 3.5 |
| | 2 | 7.1 | 5.8 | 5.6 | 5.2 |
| | 3 | 4.1 | 3.6 | 3.7 | 3.7 |
| | 6 | 3.5 | 3.2 | 3.1 | 3.3 |
| | 9 | 3.0 | 5.8 | 2.5 | 3.3 |
| | 12 | 3.0 | 2.6 | 2.8 | 2.9 |
| Binding activity by ELISA (%) | 0 | 99.0 | 104.3 | 108.8 | 101.7 |
| | 1 | 104.3 | 95.6 | 103.8 | 100.7 |
| | 2 | 130.2 | 88.2 | 108.0 | 105.1 |
| | 3 | 75.5 | 84.7 | 95.8 | 78.9 |
| | 6 | 99.6 | 98.8 | 102.0 | 94.4 |
| | 9 | 99.6 | 98.8 | 102.0 | 94.4 |
| | 12 | 98.6 | 94.4 | 106.8 | 83.4 |
| Biological activity (LDL uptake assay in HepG2 cells) | 0 | 119.6 | 101.7 | 114.1 | 122.4 |
| | 1 | 105.7 | 102.9 | 94.6 | 97.9 |
| | 2 | 110.9 | 97.3 | 96.8 | 83.5 |
| | 3 | 108.7 | 90.3 | 98.7 | 107.3 |
| | 6 | 105.6 | 84.9 | 92.3 | 108.0 |
| | 9 | 104.1 | 103.3 | 106.1 | 87.8 |
| | 12 | 108.7 | 97.7 | 97.2 | 102.4 |

Example 5. Stability of highly concentrated anti-PCSK9 antibodies at room temperature Liquid pharmaceutical formulations containing therapeutic antibodies generally require a temperature of 2-8 °C until the end of storage. Therefore, the drug requires refrigeration during the period from purchase to use. Based on the proposed dose regimen, this may result in a storage up to several weeks by the patient in the case of self-administration. Thus, drugs that do not require refrigeration show significantly increased convenience for home care circumstances and a reduction in the impact of drug quality in the event of incorrect storage, thereby reducing the complaint rates and need for monitoring the temperature deviations.

As shown in Table 9, the formulations disclosed herein (formulation no. 20) have a higher stability against protein degradation, with the resulting degradation kinetics measured at 25 °C meeting the requirements of environmental storage for up to 6 months.

**Table 9. Summary of accelerated stability study (25±2 °C)**

| Test item | Detection | Specification | Time (month) | Result |
|---|---|---|---|---|
| Purity | SEC-HPLC | Monomer content ≥ 97.0% | 0 | 99.7% |
| | | | 1 | 99.6% |
| | | | 3 | 99.3% |
| | | | 6 | 99.4% |
| | | Polymer content ≤ 3.0% | 0 | 0.3% |
| | | | 1 | 0.4% |
| | | | 3 | 0.4% |
| | | | 6 | 0.6% |
| | | Fragment content ≤ 1.0% | 0 | 0.0% |
| | | | 1 | 0.0% |
| | | | 3 | 0.3% |
| | | | 6 | 0.1% |
| | CEX-HPLC | Main peak ≥ 70.0% | 0 | 90.1% |
| | | | 1 | 83.9% |
| | | | 3 | 74.7% |
| | | | 6 | 64.6% |
| | | Acid peak ≤ 30.0% | 0 | 7.4% |
| | | | 1 | 12.2% |
| | | | 3 | 22.4% |
| | | | 6 | 32.8% |
| | | Basic peak ≤ 15.0% | 0 | 2.5% |
| | | | 1 | 3.9% |
| | | | 3 | 2.9% |
| | | | 6 | 2.6% |
| | Reduced CE-SDS | Sum of heavy chain, non-glycosylated heavy chain, and light chain ≥ 95.0% | 0 | 99.9% |
| | | | 1 | 99.8% |
| | | | 3 | 99.5% |
| | | | 6 | 98.9% |
| | Non-reduced CE-SDS | Main peak ≥ 90.0% | 0 | 97.9% |
| | | | 1 | 96.9% |
| | | | 3 | 97.9% |
| | | | 6 | 97.3% |
| Potency | Binding activity (ELISA) | 70% to 130% of reference standard | 0 | 111.0% |
| | | | 1 | 95.0% |
| | | | 3 | 88.6% |
| | | | 6 | 80.2% |
| | Biological activity (LDL uptake assay in HepG2 cells) | 70% to 130% of reference standard | 0 | 104.0% |
| | | | 1 | 94.2% |
| | | | 3 | 92.6% |
| | | | 6 | 81.1% |

### Example 6. ForteBio affinity assay (bio-layer interferometry)

ForteBio affinity assays were implemented according to the prior art. Briefly, 4 mg of JS002 (formulation no. 29) and evolocumab (140 mg/mL/vial, purchased from AMGEN, lot no. 1063135) were subjected to a 100-fold buffer exchange with phosphate buffer in a 10 KD ultrafiltration tube. After the buffer exchange, the protein content was measured by the absorbance at 280 nm, and the concentration was adjusted to 2 mg/mL. 2 mg of Sulfo-NHS-Biotin was equilibrated to room temperature and added to 300 µL of ultrapure water to give a 10 mM Biotin stock solution. 1 mL of 2 mg/mL JS002 and evolocumab were transferred to a new EP tube, and 8 µL of biotin stock solution was added according to a ratio of protein:biotin=1:6. The mixture was well mixed and incubated at room temperature for 1-2 h. After biotinylation, the samples were subjected to a 100-fold buffer exchange with phosphate buffer in a 10 KD ultrafiltration tube. After the buffer exchange, the protein content was measured by the absorbance at 280 nm, and the concentration was adjusted to 1-2 mg/mL. The biotinylated protein was aliquoted in tubes at 0.1 mL per tube, and was stored at -80 °C with no more than 1 freeze-thaw. Biotinylated JS002 and evolocumab (5 µg/mL) were conjugated with streptavidin (SA) bioprobes, equilibrated with a buffer (0.1% BSA, 0.02% Tween-20 and 1 × PBS) for 300s, and added with diluted PCSK9 at different concentrations before a 300-s association and a 1800-s dissociation. The affinity constant was calculated by the formula = k_{off}/kₒₙ.

The test results are shown in Table 10. ForteBio results show that for the binding affinity with PCSK9, JS002 is significantly superior to evolocumab sharing the same target.

**Table 10. Comparison of JS002 and Repatha affinity for human PCSK9 by ForteBio**

| | KD (M) | kon (1/Ms) | kdis (1/s) |
|---|---|---|---|
| JS002 | 2.15⁻¹¹ | 6.02E+04 | 1.30E-06 |
| Evolocumab | 6.51⁻¹⁰ | 4.92E+04 | 3.21E-05 |

### Example 7. Cellular biological activity assay for JS002 (LDL uptake assay in HepG2 cells)

In this assay, JS002 (formulation no. 29) was evaluated for the influence on uptake of LDL by HepG2 cells exposed to human PCSK9-D347Y at the cellular level, and was compared with the marketed product evolocumab (140 mg/mL/vial, purchased from AMGEN, lot no. 1063135) sharing the same target. Briefly, human liver cancer cells (HepG2; ATCC, lot no. 62591368) were plated at a density of 2.0 × 10⁴ cells/well (80 µL per well) at 37 °C, 7% CO₂, and were incubated overnight. JS002 and evolocumab were serially diluted (initial concentration: 20 µg/mL, serial 2-fold dilution). HepG2 cells were added with 10 µL of diluted antibody, incubated for half an hour, and added with 10 µL of 1 µg/mL antigen solution before another incubation for 4-6 h. Fluorescently labeled LDL (3 µg/mL) was added, and co-incubated with the cells for 16-18 h. The fluorescence signal intensity in the cells was then detected by a microplate reader.

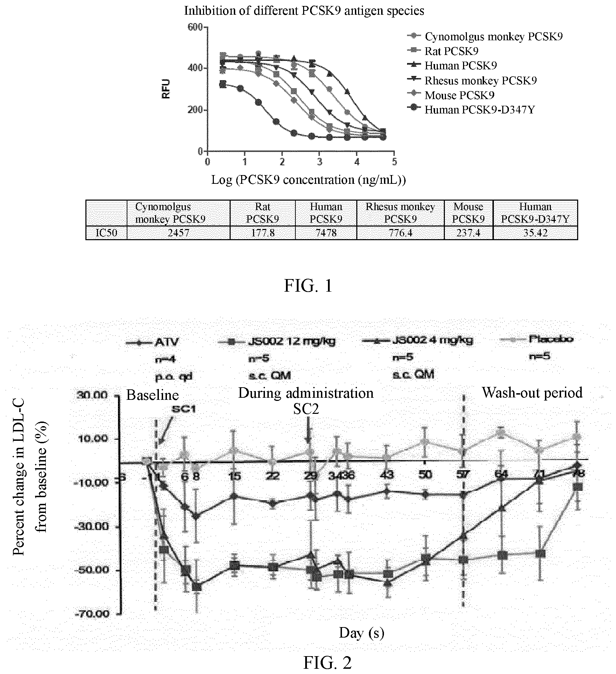

The experimental results are shown in FIG. 1, suggesting that JS002 can bind to human PCSK9-D347Y on cell surface, thus inhibiting the binding of PCSK9-D347Y to LDLR and elevating the binding and uptake of LDL by LDLR. Furthermore, JS002 (EC₅₀=92.68 ng/mL) has a significantly better effect on promoting LDL endocytosis than evolocumab (EC₅₀=151.1 ng/mL).

### Example 8. Hypolipidemic effect of JS002 in rhesus monkeys with hyperlipidemia

19 hyperlipidemic rhesus monkeys (LDL ≥ 1.3 mmol/L) were randomized into 4 groups: atorvastatin calcium group (1.2 mg/kg at weeks 1-4, 2.4 mg/kg at weeks 5-8; 4 animals), JS002 high-dose group (12 mg/kg; 5 animals), JS002 low-dose group (4 mg/kg; 5 animals) and placebo group (5 animals). The atorvastatin calcium group were orally administrated for 56 days continuously, with a wash-out period of 21 days; the JS002 high-dose group and JS002 low-dose group were administered once every 4 weeks for 2 doses, and were continuously observed for 78 days; Changes in primary efficacy endpoints (LDL-C, TC, HDL-C, ApoAL, ApoB and TG), secondary efficacy endpoint (weight) and safety endpoints (blood biochemistry, routine blood test and clinical observation) were determined regularly in the administration period and wash-out period, and JS002 (formulation no. 20) was evaluated for its hypolipidemic efficacy in rhesus monkeys with hyperlipoidemia.

The results of the experiment are shown in FIG. 2. In this study, the blood lipid level in the placebo group was stable, and the efficacy in the positive control atorvastatin calcium group was similar to previous clinical reports, suggesting that the experimental system is stable and reliable.

In the experimental system, in the high-dose group at 12 mg/kg (equivalent to a proposed clinical dose of 420 mg/70 kg in human, one subcutaneous dose every 4 weeks for a total of 2 doses), the test article JS002 demonstrated a significant decrease in LDL-C in rhesus monkeys with hyperlipidemia, and on Day 2-Day 71, the LDL-C decrease was maintained at 40%-70% compared with the baseline; for the JS002 low-dose group at 4 mg/kg (equivalent to a proposed clinical dose of 140 mg/70 kg in human, one subcutaneous dose every 4 weeks for a total of 2 doses), an LDL-C decrease by 20%-70% was maintained during Day 2-Day 57 compared with the baseline, and the LDL-C level returned during Day 57-Day 78. The intensity and duration of action in the JS002 high-dose group were superior to those of the JS002 low-dose group (FIG. 2). No obvious changes in TG, FPG, ApoAl or HDL-C were observed in the administration period; no changes in safety endpoints related to the treatment were observed.

### Example 9. Pharmacokinetics in cynomolgus monkeys

The pharmacokinetics in healthy cynomolgus monkeys after a single or multiple administrations of JS002 (formulation no. 20) at different doses were explored in this study. The grouping and regimen of the cynomolgus monkey study are shown in Table 11. Groups A, B, C and D were administered once at doses of 2 mg/kg, 10 mg/kg, 50 mg/kg and 10 mg/kg, respectively, in which groups A, B and C were administered subcutaneously and group D was administered intravenously. Group E was administered with a continuous dose of 10 mg/kg once weekly for four doses.

**Table 11: Grouping and regimen**

| Group | Test article | Route of administration (frequency) | Dosage (mg/kg) | Number of subjects |
|---|---|---|---|---|
| A | JS002 | *Sc* (1) | 2 | 8, half female and half male |
| B | JS002 | *Sc* (1) | 10 | 8, half female and half male |
| C | JS002 | *Sc* (1) | 50 | 8, half female and half male |
| D | JS002 | *I v*(1) | 10 | 8, half female and half male |
| E | JS002 | *sc* (QW×4W) | 10 | 8, half female and half male |

| | | | | |
|---|---|---|---|---|
| Note: 1. *sc*, subcutaneous administration 2. *iv*, intravenous administration 3. QW, once weekly. | | | | |

Serum samples were prepared after sampling, and the serum concentration was determined by a validated ELISA assay. The results are shown in Table 12.

**Table 12. Pharmacokinetics in cynomolgus monkeys (mean±standard deviation)**

| Parameter (unit) | A | B | C | D | E |
|---|---|---|---|---|---|
| C₀ (µg/mL) | NA | NA | NA | 267.95±87.77 | NA |
| t_{1/2} (hr) | 56.15±19.00 | 74.36±14.28 | 69.31±26.07 | 65.86±20.75 | 85.89±16.01 |
| Tₘₐₓ (hr) | 24.00±15.71 | 31.50±22.16 | 27.75±13.96 | 0.427±0.663 | 540.75±39.34 |
| Cₘₐₓ (µg/mL) | 15.44±1.99 | 107.84±31.41 | 436.50±196.90 | 266.43±75.02 | 203.11±84.48 |
| AUC₍₀₋ₜ₎ (hr·µg/mL) | 1897.13±452. 31 | 19087.95±7429.1 1 | 91249.59±26754.1 1 | 21411.41±4595.1 4 | 43699.30±20948.4 8 |
| Vd (mL/kg) | 86.08±24.49 | 62.61±23.99 | 54.97±18.13 | 44.44±12.06 | 77.89±76.91 |
| CL (mL/hr/kg) | 1.11±0.269 | 0.603±0.243 | 0.592±0.186 | 0.486±0.103 | 0.732±0.962 |
| F | NA | NA | NA | 89.15% | NA |

| | | | | | |
|---|---|---|---|---|---|
| Note: NA indicates not detected. | | | | | |

The results showed that after a single subcutaneous dose of JS002 at different doses (2, 10, 50 mg/kg), the serum drug exposure increased with the dose, suggesting a non-linear pharmacokinetic profile. Elimination half-life t_{1/2} of JS002 was 56-74 h, and effective half-life within 336 h was 50-139 h.

The bioavailability of the tested article JS002 after a single subcutaneous dose at 10 mg/kg was 89.15% in cynomolgus monkeys.

After continuous subcutaneous doses of JS002 at 10 mg/kg (1 dose weekly for 4 doses), the cynomolgus monkeys demonstrated a significantly higher exposure level compared with the single-dose group at the same dose, suggesting a significant accumulation in the body.

### Example 10. Immunotoxicity and immunogenicity in cynomolgus monkeys

In this study, the toxicity was observed in cynomolgus monkeys after four weeks of JS002 (formulation no. 20) subcutaneous administration. As per the GLP regulation of NMPA (formerly, CFDA), potential immunotoxicity and immunogenicity of the recombinant humanized anti-PCSK9 monoclonal antibody injection was evaluated. Animals were randomized into four groups, i.e., excipient control group (0 mg/kg), low-dose group (30 mg/kg), medium-dose group (100 mg/kg) and high-dose group (300 mg/kg), with 10 animals in each group, half female and half male. The drug was administered once weekly for 4 weeks, prior to a 4-week recovery period. Within the investigated dose range, no anti-drug antibody was detected in blood samples of all cynomolgus monkeys at any time points after receiving multiple administrations of excipient and different doses of JS002 (30, 100 and 300 mg/kg); both the sample positive rate and the individual positive rate were 0.0% for all groups. The test article JS002 showed low immunogenicity in cynomolgus monkeys after multiple subcutaneous administrations at different doses (30, 100 and 300 mg/kg). The immune complex assay in kidney tissues of all animals suggested no deposition of immune complex. Other immune related parameters showed no regular change or abnormality associated with the treatment, including absolute and differential WBC counts, immunoglobulin level and AIG ratio, the gross anatomy of lymph organs/tissues, weight/weight ratio of thymus and spleen, and lymphocyte subgroup distribution. The results also suggested normality in histopathology.

## Claims

1. A stable antibody formulation comprising:
(a) a buffer;
(b) a stabilizer; and
(c) an antibody or an antigen-binding fragment thereof that specifically binds to human PCSK9, wherein the antibody or the antigen-binding fragment thereof comprises HCDR1, HCDR2, HCDR3, LCDR1, LCDR2 and LCDR3, wherein:
HCDR1 has an amino acid sequence of SEQ ID NO: 1;
HCDR2 has an amino acid sequence of SEQ ID NO: 2;
HCDR3 has an amino acid sequence of SEQ ID NO: 3;
LCDR1 has an amino acid sequence of SEQ ID NO: 4;
LCDR2 has an amino acid sequence of SEQ ID NO: 5; and
LCDR3 has an amino acid sequence of SEQ ID NO: 6; and
the pH of the formulation is 5.5-6.5.

2. The antibody formulation according to claim 1, wherein the buffer is an acetate buffer or a histidine buffer.

3. The antibody formulation according to claim 2, wherein the buffer is a histidine buffer prepared by L-histidine and L-histidine monohydrochloride.

4. The antibody formulation according to claim 2, wherein the histidine buffer comprises histidine at 1-10 mM and a histidine salt at 10-20 mM; preferably, the molar ratio of histidine to the histidine salt is in the range of 1:1-1:4.

5. The antibody formulation according to claim 1, wherein the concentration of the buffer is 15-25 mM.

6. The antibody formulation according to claim 1, wherein the stabilizer is arginine or a salt thereof, sorbitol, mannitol or sucrose, or a combination of arginine or a salt thereof and sorbitol, mannitol or sucrose.

7. The antibody formulation according to claim 6, wherein the stabilizer is an arginine salt at 50-200 mM in the formulation; or the stabilizer is a mixture of an arginine salt and mannitol or sorbitol, wherein the concentration of the arginine salt in the formulation is 50-150 mM, the concentration of mannitol or sorbitol is 50-200 mM, and the total concentration of the two stabilizers is in the range of 50-250 mM.

8. The antibody formulation according to claim 1, wherein the antibody or the antigen-binding fragment thereof comprises a heavy chain variable region (VH) having an amino acid sequence of SEQ ID NO: 7 and a light chain variable region (VL) having an amino acid sequence of SEQ ID NO: 8.

9. The antibody formulation according to claim 1, wherein the antibody or the antigen-binding fragment thereof comprises a heavy chain (HC) having an amino acid sequence of SEQ ID NO: 9 and a light chain (LC) having an amino acid sequence of SEQ ID NO: 10.

10. The antibody formulation according to claim 1, wherein the antibody formulation further comprises polysorbate 20 or polysorbate 80 at about 0.01% to about 0.05%.

11. The antibody formulation according to claim 1, comprising:
(a) a histidine buffer at about 20 mM;
(b) an arginine salt at about 100-200 mM;
(c) polysorbate 20 at about 0.02%; and
(d) the antibody or the antigen-binding fragment that specifically binds to human PCSK9 at about 100 mg/mL to about 200 mg/mL.

12. The antibody formulation according to claim 1, wherein the antibody formulation is selected from antibody formulations comprising:
(A) (1) the anti-PCSK9 antibody or the antigen-binding fragment thereof at about 100 mg/mL to about 200 mg/mL; (2) a histidine buffer at about 10-50 mM, about pH 5.5-6.5; (3) an arginine salt at about 50 mM to about 200 mM; and (4) a nonionic surfactant at about 0% to about 0.1%;
(B) (1) the anti-PCSK9 antibody or the antigen-binding fragment thereof at about 150±10 mg/mL; (2) a histidine buffer at about 20 mM, about pH 5.5-6.5; (3) an arginine salt at about 60±5 mM, 130±5 mM or 160±5 mM; and (4) a nonionic surfactant at about 0% to about 0.1%;
(C) (1) the anti-PCSK9 antibody or the antigen-binding fragment thereof at about 150±10 mg/mL, wherein the antibody comprises a heavy chain variable region (VH) having an amino acid sequence of SEQ ID NO: 7 and a light chain variable region (VL) having an amino acid sequence of SEQ ID NO: 8; (2) a histidine buffer at about 20 mM, about pH 5.5-6.5; (3) arginine at about 160±5 mM; and (4) polysorbate 20 at about 0.02%;
(D) (1) the anti-PCSK9 antibody or the antigen-binding fragment thereof at about 150±10 mg/mL, wherein the antibody is a full-length antibody comprising a heavy chain having an amino acid sequence of SEQ ID NO: 9 and a light chain having an amino acid sequence of SEQ ID NO: 10; (2) a histidine buffer at about 20 mM, about pH 5.5-6.5; (3) arginine at about 160±5 mM; and (4) polysorbate 20 at about 0.02%;
(E) (1) the anti-PCSK9 antibody or the antigen-binding fragment thereof at 150 mg/mL; (2) a histidine buffer at 20 mM; (3) arginine hydrochloride at 160 mM; and (4) polysorbate 20 at 0.02%;
(F) (1) the anti-PCSK9 antibody or the antigen-binding fragment thereof at 150 mg/mL; (2) a histidine buffer at 20 mM; (3) arginine hydrochloride at 120 mM and mannitol at 60 mM; and (4) polysorbate 20 at 0.02%; or
(G) (1) the anti-PCSK9 antibody or the antigen-binding fragment thereof at 150 mg/mL; (2) a histidine buffer at 20 mM; (3) arginine hydrochloride at 60 mM and mannitol at 160 mM; and (4) polysorbate 20 at 0.02%.

13. A delivery device comprising the antibody formulation according to any of claims 1-12.

14. A pre-filled syringe comprising the antibody formulation according to any of claims 1-12.

15. Use of the antibody formulation according to any of claims 1-12, the delivery device according to claim 13, or the pre-filled syringe according to claim 14 in treating, preventing or improving any diseases associated with PCSK9 activity.
